(19) Europäisches Patentamt
European Patent Office
Office européen des brevets

(11) EP 3 320 850 A1

(12) EUROPEAN PATENT APPLICATION

(43) Date of publication:
16.05.2018 Bulletin 2018/20

(51) Int Cl.:
A61B 8/08 (2006.01)

(21) Application number: 17169053.0

(22) Date of filing: 02.05.2017

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD

(30) Priority: 15.11.2016 KR 20160152230

(71) Applicant: Samsung Medison Co., Ltd.
Hongcheon-gun, Gangwon-do, 25108 (KR)

(72) Inventors:
• Yang, Sun-mo
Gangwon-do (KR)
• Shin, Seong-chul
Gangwon-do (KR)
• Ahn, Dong-il
Gangwon-do (KR)

(74) Representative: Hylarides, Paul Jacques
Arnold & Siedsma
Bezuidenhoutseweg 57
2594 AC Den Haag (NL)

(54) ULTRASOUND DIAGNOSIS APPARATUS AND METHOD OF CONTROLLING THE SAME

(57) Provided is an ultrasound diagnosis apparatus including: an ultrasound probe configured to acquire ultrasound image data by transmitting ultrasound signals to an object and detecting reflected signals corresponding to the transmitted ultrasound signals; a processor configured to obtain an elastography image based on the ultrasound image data; and a display configured to display the elastography image. The processor is further configured to determine, based on the elastography image, a region of interest (ROI) for acquiring shear wave elasticity measurement data and a focusing position of a focus beam for performing shear wave elasticity measurement, and the ultrasound probe is further configured to emit the focus beam onto the focusing position and acquire shear wave elasticity measurement data.

FIG. 11

START

ACQUIRE ULTRASOUND IMAGE DATA WITH RESPECT TO OBJECT — S1102

OBTAIN ELASTOGRAPHY IMAGE BASED ON ULTRASOUND IMAGE DATA — S1104

DISPLAY ELASTOGRAPHY IMAGE — S1106

DETERMINE ROI FOR ACQUIRING SHEAR WAVE ELASTICITY MEASUREMENT DATA AND FOCUSING POSITION OF FOCUS BEAM FOR SHEAR WAVE ELASTICITY MEASUREMENT — S1108

ACQUIRE SHEAR WAVE ELASTICITY MEASUREMENT DATA BY EMITTING FOCUS BEAM ONTO FOCUSING POSITION — S1110

END

EP 3 320 850 A1

**Description**

BACKGROUND

1. Field

The present disclosure relates to ultrasound diagnosis apparatuses, methods of controlling the ultrasound diagnosis apparatuses, and computer-readable recording media having recorded thereon program code for performing the methods of controlling the ultrasound diagnosis apparatuses.

2. Description of the Related Art

Ultrasound diagnostic apparatuses transmit ultrasound signals generated by transducers of a probe to an object and detect information about signals reflected from the object, thereby obtaining at least one image of an internal part, for example, soft tissue or blood flow, of the object.

Such ultrasound diagnosis apparatuses provide high stability, display images in real time, and are safe due to no radiation exposure, compared to X-ray diagnosis apparatuses. Therefore, an ultrasound diagnosis apparatus is widely used together with other types of imaging diagnosis devices.

SUMMARY

Provided are methods and apparatuses for determining, during shear wave elasticity measurement, a region of interest (ROI) where shear wave elasticity measurement data is to be acquired and a focusing position of a focus beam with respect to the ROI.

Provided are methods and apparatuses for determining an ROI of an object based on elasticity data obtained from an elastography image and then a focusing position of a focus beam with respect to the determined ROI by providing the elastography image during shear wave elasticity measurement.

Provided are methods and apparatuses for acquiring shear wave elasticity estimation data from a region other than an ROI based on elasticity data from an elastography image and acquired shear wave elasticity measurement data.

Provided are methods and apparatuses that are used to increase a success rate of acquisition of a shear wave elastography image by preventing the failure of such acquisition due to emission of a focus beam onto an obstacle by determining a focusing position of a focus beam based on an elastography image.

Additional aspects will be set forth in part in the description which follows and, in part, will be apparent from the description, or may be learned by practice of the presented embodiments.

According to an aspect of an embodiment, an ultrasound diagnosis apparatus includes: an ultrasound probe configured to acquire ultrasound image data by transmitting ultrasound signals to an object and detecting reflected signals corresponding to the transmitted ultrasound signals; a processor configured to obtain an elastography image based on the ultrasound image data; and a display configured to display the elastography image. The processor is further configured to determine, based on the elastography image, an ROI for acquiring shear wave elasticity measurement data and a focusing position of a focus beam for performing shear wave elasticity measurement, and the ultrasound probe is further configured to emit the focus beam onto the focusing position and acquire shear wave elasticity measurement data.

The processor is further configured to calculate an elasticity value according to a position based on the elastography image and determine a region having the elasticity value less than a first reference value as being the ROI.

The ultrasound diagnosis apparatus may further include a user input interface configured to receive, when there are a plurality of regions having elasticity values less than the first reference value, a user input for selecting one of the plurality of regions, and the processor is further configured to determine the ROI based on the user input.

The processor is further configured to determine a scan line for the focus beam, which has a first interval away from the ROI, and determine the focusing position of the focus beam based on the ROI.

The processor is further configured to determine a plurality of scan lines, each scan line having an interval that is within a first range away from the ROI, and the display is further configured to display the determined plurality of scan lines. The ultrasound diagnosis apparatus may further include a user input interface configured to receive a user input for selecting one of the plurality of scan lines as a scan line for the focus beam.

The display is further configured to display the acquired shear wave elasticity measurement data.

The processor is further configured to obtain shear wave elasticity estimation data in a region other than the ROI in the elastography image based on elasticity data obtained from the elastography image and the shear wave elasticity measurement data acquired with respect to the ROI.

The display is further configured to display the shear wave elasticity measurement data and the shear wave elasticity estimation data.

The processor is further configured to determine a forbidden focus beam emission region based on the elastography image and determine the focusing position in such a manner as to prevent emission of the focus beam onto the forbidden focus beam emission region.

According to an aspect of another embodiment, a method of controlling an ultrasound diagnosis apparatus includes: acquiring ultrasound image data by transmitting ultrasound signals to an object and detecting reflected signals corresponding to the transmitted ultrasound signals; obtaining an elastography image based on the ultrasound image data; displaying the elastography image; determining, based on the elastography image, a region of interest (ROI) for acquiring shear wave elasticity measurement data and a focusing position of a focus beam for performing shear wave elasticity measurement; and emitting the focus beam onto the focusing position and acquiring shear wave elasticity measurement data.

The method further comprises receiving, when there are a plurality of regions having elasticity values less than the first reference value, a user input for selecting one of the plurality of regions, and the processor is further configured to determine the ROI based on the user input.

The determining of the focusing position comprises determining a plurality of scan lines, each scan line having an interval that is within a first range away from the ROI, displaying the determined plurality of scan lines, and receiving a user input for selecting one of the plurality of scan lines as a scan line for the focus beam.

The method further comprises displaying the shear wave elasticity measurement data and the shear wave elasticity estimation data.

The determining of the focusing position of the focus beam comprises determining a forbidden focus beam emission region based on the elastography image and determining the focusing position in such a manner as to prevent emission of the focus beam onto the forbidden focus beam emission region.

According to an aspect of another embodiment, a non-transitory computer-readable recording medium has recorded thereon computer program code for performing the method of controlling an ultrasound diagnosis apparatus.

BRIEF DESCRIPTION OF THE DRAWINGS

These and/or other aspects will become apparent and more readily appreciated from the following description of the embodiments, taken in conjunction with the accompanying drawings in which:

FIG. 1 is a block diagram illustrating an ultrasound diagnosis apparatus according to an exemplary embodiment;
FIGS. 2A, 2B, and 2C are diagrams respectively illustrating an ultrasound diagnosis apparatus according to an exemplary embodiment;
FIG. 3 is a block diagram of a structure of an ultrasound diagnosis apparatus according to an embodiment;
FIG. 4 is a block diagram of a structure of an ultrasound diagnosis apparatus according to another embodiment;
FIG. 5 illustrates a process of obtaining an elastography image by inducing a displacement in an object, according to an embodiment;
FIG. 6A illustrates a process of generating shear waves in an object according to an embodiment, and FIG. 6B illustrates propagation of shear waves according to an embodiment;
FIGS. 7A and 7B images illustrating a process of determining a region of interest (ROI) for shear wave elasticity measurement based on elasticity data from an elastography image, according to an embodiment;
FIG. 8 illustrates a view showing an elastography image displayed on a display, according to an embodiment;
FIG. 9 illustrates a process of determining, based on an ROI, a focus beam scan line for performing shear wave elasticity measurement, according to an embodiment;
FIG. 10 illustrates a process of acquiring shear wave elasticity estimation data based on shear wave elasticity measurement data obtained via shear wave elasticity measurement; and
FIG. 11 is a flowchart of a method of controlling an ultrasound diagnosis apparatus, according to an embodiment.

DETAILED DESCRIPTION

Certain exemplary embodiments are described in greater detail below with reference to the accompanying drawings.

In the following description, the same drawing reference numerals are used for the same elements even in different drawings. The matters defined in the description, such as detailed construction and elements, are provided to assist in a comprehensive understanding of exemplary embodiments. Thus, it is apparent that exemplary embodiments can be carried out without those specifically defined matters. Also, well-known functions or constructions are not described in detail since they would obscure exemplary embodiments with unnecessary detail.

Terms such as "part" and "portion" used herein denote those that may be embodied by software or hardware.

According to exemplary embodiments, a plurality of parts or portions may be embodied by a single unit or element, or a single part or portion may include a plurality of elements. Expressions such as "at least one of," when preceding a list of elements, modify the entire list of elements and do not modify the individual elements of the list.

In exemplary embodiments, an image may include any medical image acquired by various medical imaging apparatuses such as a magnetic resonance imaging (MRI) apparatus, a computed tomography (CT) apparatus, an ultrasound imaging apparatus, or an X-ray apparatus.

Also, in the present specification, an "object", which is a thing to be imaged, may include a human, an animal, or a part thereof. For example, an object may include a part of a human, that is, an organ or a tissue, or a phantom.

Throughout the specification, an ultrasound image refers to an image of an object processed based on ultrasound signals transmitted to the object and reflected therefrom.

In the present specification, a region of interest (ROI) may include a region having a specific area as well as a point corresponding to a specific position in an ultrasound image.

Hereinafter, embodiments will be described in detail with reference to the accompanying drawings.

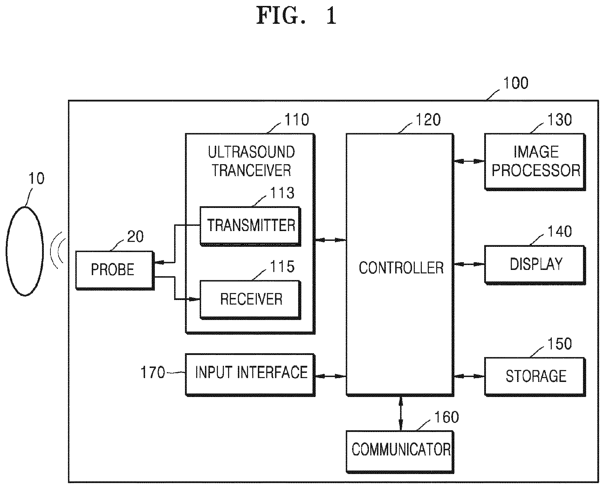

FIG. 1 is a block diagram illustrating a configuration of an ultrasound diagnosis apparatus 100, i.e., a diagnostic apparatus, according to an exemplary embodiment.

Referring to FIG. 1, the ultrasound diagnosis apparatus 100 may include a probe 20, an ultrasound transceiver 110, a controller 120, an image processor 130, one or more displays 140, a storage 150, e.g., a memory, a communicator 160, i.e., a communication device or an interface, and an input interface 170.

The ultrasound diagnosis apparatus 100 may be of a cart-type or a portable-type ultrasound diagnosis apparatus that is portable, moveable, mobile, or hand-held. Examples of the portable-type ultrasound diagnosis apparatus 100 may include a smart phone, a laptop computer, a personal digital assistant (PDA), and a tablet personal computer (PC), each of which may include a probe and a software application, but embodiments are not limited thereto.

The probe 20 may include a plurality of transducers. The plurality of transducers may transmit ultrasound signals to an object 10 in response to transmitting signals received by the probe 20, from a transmitter 113. The plurality of transducers may receive ultrasound signals reflected from the object 10 to generate reception signals. In addition, the probe 20 and the ultrasound diagnosis apparatus 100 may be formed in one body (e.g., disposed in a single housing), or the probe 20 and the ultrasound diagnosis apparatus 100 may be formed separately (e.g., disposed separately in separate housings) but linked wirelessly or via wires. In addition, the ultrasound diagnosis apparatus 100 may include one or more probes 20 according to embodiments.

The controller 120 may control the transmitter 113 for the transmitter 113 to generate transmitting signals to be applied to each of the plurality of transducers based on a position and a focal point of the plurality of transducers included in the probe 20.

The controller 120 may control the ultrasound receiver 115 to generate ultrasound data by converting reception signals received from the probe 20 from analogue to digital signals and summing the reception signals converted into digital form, based on a position and a focal point of the plurality of transducers.

The image processor 130 may generate an ultrasound image by using ultrasound data generated from the ultrasound receiver 115.

The display 140 may display a generated ultrasound image and various pieces of information processed by the ultrasound diagnosis apparatus 100. The ultrasound diagnosis apparatus 100 may include two or more displays 140 according to the present exemplary embodiment. The display 140 may include a touch screen in combination with a touch panel.

The controller 120 may control the operations of the ultrasound diagnosis apparatus 100 and flow of signals between the internal elements of the ultrasound diagnosis apparatus 100. The controller 120 may include a memory for storing a program or data to perform functions of the ultrasound diagnosis apparatus 100 and a processor and/or a microprocessor (not shown) for processing the program or data. For example, the controller 120 may control the operation of the ultrasound diagnosis apparatus 100 by receiving a control signal from the input interface 170 or an external apparatus.

The ultrasound diagnosis apparatus 100 may include the communicator 160 and may be connected to external apparatuses, for example, servers, medical apparatuses, and portable devices such as smart phones, tablet personal computers (PCs), wearable devices, etc., via the communicator 160.

The communicator 160 may include at least one element capable of communicating with the external apparatuses. For example, the communicator 160 may include at least one among a short-range communication module, a wired communication module, and a wireless communication module.

The communicator 160 may receive a control signal and data from an external apparatus and transmit the received control signal to the controller 120 so that the controller 120 may control the ultrasound diagnosis apparatus 100 in response to the received control signal.

The controller 120 may transmit a control signal to the external apparatus via the communicator 160 so that the external apparatus may be controlled in response to the control signal of the controller 120.

For example, the external apparatus connected to the ultrasound diagnosis apparatus 100 may process the data of the external apparatus in response to the control signal of the controller 120 received via the communicator 160.

A program for controlling the ultrasound diagnosis apparatus 100 may be installed in the external apparatus. The program may include command languages to perform part of operation of the controller 120 or the entire operation of the controller 120.

The program may be pre-installed in the external apparatus or may be installed by a user of the external apparatus by downloading the program from a server that provides applications. The server that provides applications may include a recording medium where the program is stored.

The storage 150 may store various data or programs for driving and controlling the ultrasound diagnosis apparatus 100, input and/or output ultrasound data, ultrasound images, applications, etc.

The input interface 170 may receive a user's input to control the ultrasound diagnosis apparatus 100 and may include a keyboard, button, keypad, mouse, trackball, jog switch, knob, a touchpad, a touch screen, a microphone, a motion input means, a biometrics input means, etc. For example, the user's input may include inputs for manipulating buttons, keypads, mice, trackballs, jog switches, or knobs, inputs for touching a touchpad or a touch screen, a voice input, a motion input, and a bioinformation input, for example, iris recognition or fingerprint recognition, but an exemplary embodiment is not limited thereto.

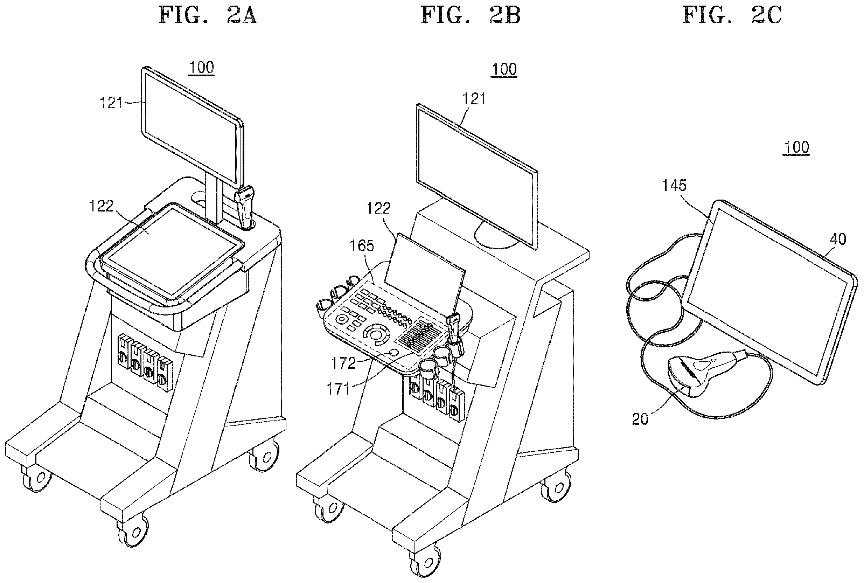

An example of the ultrasound diagnosis apparatus 100 according to the present exemplary embodiment is described below with reference to FIGS. 2A, 2B, and 2C.

FIGS. 2A, 2B, and 2C are diagrams illustrating ultrasound diagnosis apparatus according to an exemplary embodiment.

Referring to FIGS. 2A and 2B, the ultrasound diagnosis apparatus 100 may include a main display 121 and a sub-display 122. At least one among the main display 121 and the sub-display 122 may include a touch screen. The main display 121 and the sub-display 122 may display ultrasound images and/or various information processed by the ultrasound diagnosis apparatus 100. The main display 121 and the sub-display 122 may provide graphical user interfaces (GUI), thereby receiving user's inputs of data to control the ultrasound diagnosis apparatus 100. For example, the main display 121 may display an ultrasound image and the sub-display 122 may display a control panel to control display of the ultrasound image as a GUI. The sub-display 122 may receive an input of data to control the display of an image through the control panel displayed as a GUI. The ultrasound diagnosis apparatus 100 may control the display of the ultrasound image on the main display 121 by using the input control data.

Referring to FIG. 2B, the ultrasound diagnosis apparatus 100 may include a control panel 165. The control panel 165 may include buttons, trackballs, jog switches, or knobs, and may receive data to control the ultrasound diagnosis apparatus 100 from the user. For example, the control panel 165 may include a time gain compensation (TGC) button 171 and a freeze button 172. The TGC button 171 is to set a TGC value for each depth of an ultrasound image. Also, when an input of the freeze button 172 is detected during scanning an ultrasound image, the ultrasound diagnosis apparatus 100 may keep displaying a frame image at that time point.

The buttons, trackballs, jog switches, and knobs included in the control panel 165 may be provided as a GUI to the main display 121 or the sub-display 122.

Referring to FIG. 2C, the ultrasound diagnosis apparatus 100 may include a portable device. An example of the portable ultrasound diagnosis apparatus 100 may include, for example, smart phones including probes and applications, laptop computers, personal digital assistants (PDAs), or tablet PCs, but an exemplary embodiment is not limited thereto.

The ultrasound diagnosis apparatus 100 may include the probe 20 and a main body 40. The probe 20 may be connected to one side of the main body 40 by wire or wirelessly. The main body 40 may include a touch screen 145. The touch screen 145 may display an ultrasound image, various pieces of information processed by the ultrasound diagnosis apparatus 100, and a GUI.

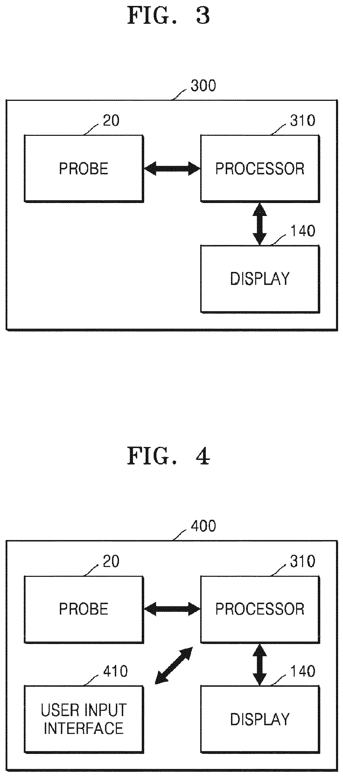

FIG. 3 is a block diagram of a structure of an ultrasound diagnosis apparatus 300 according to an embodiment.

Referring to FIG. 3, the ultrasound diagnosis apparatus 300 according to the present embodiment includes a probe 20, a processor 310, and a display 140.

The processor 310 shown in FIG. 3 may correspond to at least one of the image processor 130 and the controller 120 described with reference to FIG. 1 or a combination thereof. Furthermore, the processor 310 may include one or more processors (not shown). According to an embodiment, some of the components of the ultrasound diagnosis apparatus 100 of FIG. 1 may be included in the ultrasound diagnosis apparatus 300 of FIG. 3.

The probe 20 transmits ultrasound waves to an object and detects echo signals reflected from the object. The probe 20 may include a plurality of transducer elements. The probe 20 may be implemented as a wired or wireless probe. Furthermore, the probe 20 may include a one-dimensional (1D) transducer array, a portable 1D transducer array, or a two-dimensional (2D) transducer array.

According to an embodiment, the probe 20 may induce a displacement in tissue of the object by transmitting a focused beam to the object. For example, a beamformer may adjust delays for a specific number of transducer elements to generate a focused beam that is transmitted to the object. Furthermore, after transmitting the focused beam to the object, the probe 20 may detect echo signals from the object and acquire image data necessary for generating an elastography image. According to an embodiment, the probe 20 may use low frequency signals for transmission of a focused beam, compared to transmission of plane waves.

Ultrasound waves transmitted to generate shear waves that propagate into the object are referred to as a "focus beam," and a focal point to which the focus beam is focused is referred to as a "focusing position." Furthermore, an elasticity mode ultrasound image is hereinafter referred to as an "elastography image." For example, the elasticity mode ultrasound image may be obtained when compression is applied to the object via the probe 20.

The processor 310 controls all operations of the ultrasound diagnosis apparatus 300 and processes data and signals. The processor 310 may include one or more processors. According to an embodiment, the processor 310 may include an image processor (not shown) and a controller (not shown). The processor 310 may be implemented as software modules created by executing program code stored in a storage (not shown).

When the ultrasound diagnosis apparatus 300 operates in an elasticity mode, the processor 310 calculates a displacement of tissue of the object from an obtained ultrasound image.

According to an embodiment, the displacement may be calculated by comparing a plurality of ultrasound images obtained before and after compression is applied to the object. Furthermore, the displacement may be determined by performing auto-correlation or cross-correlation on ultrasound images obtained before and after movement of the object. According to another embodiment, the displacement may be calculated by using a differential image between ultrasound images obtained before and after movement of the object or by differentiating an obtained ultrasound image with respect to time.

The processor 310 calculates strain by differentiating the resulting displacement in a depth direction.

According to an embodiment, the processor 310 may include modules such as a displacement calculator and a strain calculator.

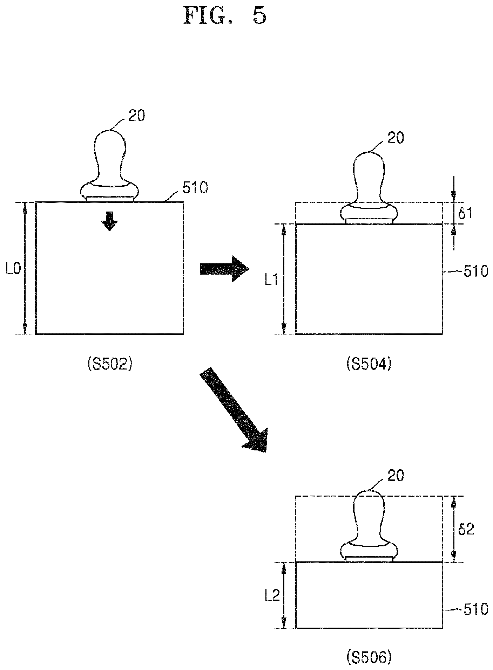

The processor 310 may obtain an elastography image based on the calculated strain. Elastic properties and elasticity values of the tissue of the object may be expressed as strain. Strain may be defined as a ratio of a displacement of tissue to a total length of the tissue before the displacement occurs. Obtaining strain by inducing a displacement in tissue of an object will be described in more detail below with reference to FIG. 5.

According to an embodiment, the processor 310 may obtain, based on the calculated strain, an elastography image showing a point having a low elasticity value, such as a tumor, in a red color and a point having a high elasticity value in a blue color. However, colors for elasticity values on the elastography image are not limited to red and blue colors and may be set in various other ways according to embodiments. For example, colors for elasticity values may be preset, or be set according to a user input.

According to an embodiment, the processor 310 may obtain an elastography image based on an ultrasound image of the object prestored in the storage.

In one embodiment, the processor 310 may control the display 140 to display an elastography image of the object prestored in the storage. When a probe scan position when the elastography image is obtained is different from a current probe scan position, the processor 310 may control the display 140 to display a graphical indicator, such as arrows, shapes, and text, which indicates adjustment of a position of the probe 20.

The processor 310 determines an ROI where shear wave elasticity measurement data is to be acquired, based on an elastography image. The processor 310 may set a region having an elasticity less than a first reference value as the ROI. According to an embodiment, the processor 310 may determine, based on the elastography image, a region having strain less than a specific value as being an ROI for acquiring shear wave elasticity measurement data.

The first reference value for determining the ROI may be acquired from a server connected to the ultrasound diagnosis apparatus 300 by wire or wirelessly or may be a value stored in the storage of the ultrasound diagnosis apparatus 300. Furthermore, the first reference value may be input by a user.

According to an embodiment, if there are a plurality of regions having strains less than the first reference value, the processor 310 may determine one region that is selected among the plurality of regions according to a user input as being the ROI.

According to another embodiment, if there are a plurality of regions having strains less than the first reference value, the processor 310 may determine only one region among the plurality of regions as being the ROI according to a preset criterion. For example, according to the preset criterion, a region including a point having the lowest strain, a point included in a preset body part or region, or the like may be determined as the ROI. The body part may be distinguished by segmenting a B-mode image.

According to an embodiment, the processor 310 may control the display 140 to display an elastography image. For example, the processor 310 may control the display 140 to display an elastography image together with a B-mode image. The B-mode image and the elastography image may be displayed in separate regions or overlapping each other.

According to an embodiment, the processor 310 may provide a graphical user interface (GUI) via which the user selects an ROI on a screen where an elastography image and a B-mode image are displayed together. The processor 310 may determine a specific region including points selected according to a user input as being the ROI. The user may determine a region in an object which requires shear wave elasticity measurement based on a displayed elastography image and select the determined region as the ROI. For example, the processor 310 may obtain an elastography image represented by a color bar including colors that are distinguished according to strains of the tissue. Furthermore, the processor 310 may control the display 140 to display the obtained elastography image. The user may determine, based on the displayed elastography image, a region indicated in a different color from the other regions as being a region to be a lesion and perform an input for selecting the determined region as the ROI. The processor 310 may determine the region selected according to the user input as being the ROI.

The processor 310 determines a focus beam scan line and a focusing position for performing shear wave elasticity measurement based on the ROI.

According to an embodiment, the processor 310 may determine a focus beam scan line along which a focus beam is to be emitted in order to acquire shear wave elasticity measurement data with respect to the ROI.

When a focus beam is emitted onto the object, a displacement of the object may be induced at a focusing position where the focus beam is focused. Due to the displacement of the object, shear waves propagating in a vertical direction of the displacement are generated at a position where the displacement occurs. If an offset between the focusing position and the ROI is extremely short, it is difficult to accurately measure shear wave elasticity data in the ROI since curved-shaped shear waves propagate into the ROI. Otherwise, if the offset between the focusing position and the ROI is extremely long, the magnitude of the shear waves may not be sufficient to measure shear wave elasticity data in the ROI due to their attenuation. Thus, the processor 310 may determine a first interval corresponding to an offset between the focusing position and the ROI based on a variation in shear waves generated at the focusing position, along a propagation direction. For example, the processor 310 may calculate the first interval by using Equation (1) below based on the variation in the direction of propagation:

$$\varepsilon = \frac{\partial^2 u_z}{\partial y^2} / \left( \frac{\partial^2 u_z}{\partial x^2} + \frac{\partial^2 u_z}{\partial z^2} \right) < Threshold \qquad \dots (1)$$

where $\varepsilon$ is an error rate, $u_z$ is a displacement of shear waves in the z-axis direction (the direction of propagation), and a Threshold is a preset threshold.

In Equation (1), the error rate $\varepsilon$ is proportional to a variation in shear waves along the y-axis (depth) direction and is inversely proportional to the sum of variations in the x- and z-axis directions. The processor 310 may determine a displacement of shear waves in the z-axis direction so that an error rate is less than a preset threshold and determine a value greater than an offset between the displacement and an ROI as being the first interval.

According to an embodiment, the first interval corresponding to the offset between the focusing position and the ROI may be acquired from a server connected to the ultrasound diagnosis apparatus 300 by wire or wirelessly or may be a value stored in the storage of the ultrasound diagnosis apparatus 300. Furthermore, the first interval may be input by the user.

According to an embodiment, the processor 310 may determine a focus beam scan line having the first interval away from the ROI. For example, the processor 310 may determine a straight line in a depth direction, which is the first interval away from the ROI, as being the focus beam scan line. The processor 310 may determine two straight lines in the depth direction, which are first intervals away from the ROI rightward and leftward, respectively. The processor 310 may determine one of the two straight lines as being a focus beam scan line according to a preset criterion. For example, the processor 310 may determine, as a focus beam scan line, a straight line in the depth direction, which is spaced rightward apart from the ROI by the first interval. Alternatively, the processor 310 may determine, as a focus beam scan line, a straight line in the depth direction, which is spaced leftward apart from the ROI by the first interval. Furthermore, the processor 310 may determine, as a focus beam scan line, a straight line selected based on a user input for selecting one of the two straight lines.

According to an embodiment, the processor 310 may determine a plurality of scan lines, each scan line having an interval that is within a first range away from the ROI. Furthermore, the processor 310 may control the display 140 to display the determined plurality of scan lines. The processor 310 may determine a focus beam scan line based on a user input for selecting one of the plurality of scan lines.

The processor 310 determines a focusing position of a focus beam based on the determined focus beam scan line and the determined ROI.

According to an embodiment, the processor 310 may determine a point on the focus beam scan line, which is located at the same depth as the ROI, as the focusing position of the focus beam.

According to an embodiment, the processor 310 may determine, as the focusing position, one point from among a point on a focus beam scan line that is located at the same depth as the ROI and its adjacent points on the focus beam scan line.

According to an embodiment, the processor 310 may determine a forbidden focus beam emission region excluding a focusing position of a focus beam. For example, the processor 310 may determine a region where a lesion such as a tumor is located as a forbidden focus beam emission region. When a focus beam having relatively large energy is emitted onto the region, exposure to the focus beam may harm the object (patient). The processor 310 may determine, based on an elastography image, a region having strain less than a specific value as being a forbidden focus beam emission region. A second reference value, which is the specific value used as a reference in determining a forbidden focus beam emission region, may be less than the above-described first reference value for determining an ROI. The processor 310 may determine, as a focusing position, a point that is not included in the forbidden focus beam emission region from among a point on a focus beam scan line that is located at the same depth as the ROI and its adjacent points on the focus beam scan line.

According to an embodiment, the processor 310 may determine a focusing position by excluding a hard region detected in an elastography image. For example, the hard region may include a region with low elasticity and a region including bone. Furthermore, according to an embodiment, the processor 310 may determine a focusing position by excluding a forbidden focus beam emission region.

The processor 310 controls the probe 20 so that a focus beam is emitted onto the determined focusing position and acquires elasticity in the ROI based on received echo signals.

According to an embodiment, the processor 310 may calculate elasticity in the ROI by using Equation (2) below:

$$\rho \frac{\partial^2 u_z}{\partial t^2} = \mu(x, y, z)\left(\frac{\partial^2 u_z}{\partial x^2} + \frac{\partial^2 u_z}{\partial y^2} + \frac{\partial^2 u_z}{\partial z^2}\right) \qquad \dots (2)$$

where p is a density of a medium, $\mu$ is elasticity, $u_z$ is a displacement of shear waves in a direction of propagation (the z-axis direction), and the y-axis direction is a depth direction in which a beam is emitted.

In Equation (2), when the object is a human body, p may represent a density of human tissue. Furthermore, since a shear wave variation in the ROI along the x-axis direction is sufficiently small (i.e., so that shear waves travel in straight lines), $\frac{\partial^2 u_z}{\partial x^2}$ is assumed to be 0.

Elasticity in the ROI obtained via shear wave elasticity measurement is hereinafter referred to as "shear wave elasticity measurement data."

According to an embodiment, the processor 310 may perform shear wave elasticity measurements on a focusing position a plurality of times and acquire shear wave elasticity measurement data by averaging the resulting data.

According to an embodiment, the processor 310 may estimate elasticity in a region other than the ROI in the elastography image based on an elasticity value (e.g., a strain) in the elastography image and the acquired shear wave elasticity measurement data. The elasticity estimated in the region other than the ROI in the elastography image is hereinafter referred to as "shear wave elasticity estimation data."

According to an embodiment, the processor 310 may calculate a value of shear wave elasticity estimation data in a region other than an ROI by using Equation (3) below:

$$y_r = \left(\frac{x_r}{X_R}\right) \times Y_R \qquad \dots (3)$$

where $X_R$ is an elasticity value of an ROI in an elastography image, $x_r$ is a value of shear wave elasticity measurement data, $Y_R$ is an elasticity data value of a specific region in the elastography image from among regions other than the ROI, and $y_r$ is a value of shear wave elasticity estimation data in the specific region.

In an embodiment, the processor 310 may obtain a shear wave elastography image based on acquired shear wave elasticity measurement data and shear wave elasticity estimation data. For example, the processor 310 may obtain a shear wave elastography image represented by a color bar including colors that are distinguished according to values of shear wave elasticity measurement data and shear wave elasticity estimation data. Furthermore, the processor 310 may control the display 140 to display the obtained shear wave elastography image.

The display 140 displays an operating status of the ultrasound diagnosis apparatus 300, an ultrasound image, a user interface screen, etc., based on a control signal output from the processor 310.

According to an embodiment, the display 140 may display an obtained elastography image.

According to an embodiment, the display 140 may display an elastography image in such a manner as to overlay a B-mode image.

According to an embodiment, the display 140 may display an elastography image in a first area on a screen and a B-mode image in a second area thereon that is distinguished from the first area.

FIG. 4 is a block diagram of a structure of an ultrasound diagnosis apparatus 400 according to another embodiment.

Referring to FIG. 4, unlike the ultrasound diagnosis apparatus 300 of FIG. 3, the ultrasound diagnosis apparatus 400 according to the present embodiment may further include a user input interface 410. The user input interface 410 may correspond to the input interface 170 described with reference to FIG. 1.

According to an embodiment, the user input interface 410 may receive a user input for selecting one region from among a plurality of regions determined by the processor 310 as an ROI for acquiring shear wave elasticity measurement data.

According to an embodiment, the user input interface 410 may receive a user input for selecting, as a focus beam scan line, one straight line from among a plurality of straight lines determined by the processor 310.

In an embodiment, the user input interface 410 may receive a user input for selecting a specific region for acquiring shear wave elasticity estimation data from among regions other than an ROI in an elastography image.

FIG. 5 illustrates a process of obtaining an elastography image by inducing a displacement in an object, according to an embodiment

In order to obtain an elastography image, compression is applied to an object 510 to induce a displacement of the object 510. For example, as shown in operations S502 and S504 of FIG. 5, a displacement $\delta$ 1 may be induced in the object 510 by applying compression to the object 510 via a probe 20. When the displacement $\delta$ 1 is induced in the object 510, a length of the object 510 may decrease in a direction along which the compression is applied. For example, as shown in operation S504 of FIG. 5, a height of the object 510 may decrease from L0 to L1 (=L0-$\delta$ 1) by the displacement $\delta$ 1. A status of tissue may be diagnosed using the properties that a displacement may vary according to the status of tissue when compression is applied to the object 510 in this way. Thus, the user may observe the status of tissue based on an elastography image captured when the displacement is induced in the object 510.

The user may measure strain that is one of the elastic properties of tissue by using an elastography image. The strain may be defined as a ratio of a displacement of tissue of an object to a total length of the tissue before the displacement occurs. For example, if a length of the tissue of the object decreases from L0 (S502) to L1 (S504) by the displacement $\delta$ 1, the strain may be expressed as $\delta$ 1/ L0. Strain may vary depending on the status of tissue of the object. For example, In operation S506, since a ratio of displacement $\delta$ 2 of tissue to a total length L0 of the tissue is higher than the ratio observed in operation S504, the tissue may have a higher strain than in operation S504.

To obtain an elastography image by inducing a displacement of an object in this way, appropriate pressure needs to be applied. As a simple method, the user of the ultrasound diagnosis apparatus (300 of FIG. 3) may apply compression to the object 510 with the probe 20. When the user of the ultrasound diagnosis apparatus 300 applies external pressure to the object 510 by pressing the object 510 with the probe 20, obtaining an elastography may be unsuccessful since the magnitude of the external pressure varies greatly according to users. Furthermore, use of an elastography image has a limitation that only a relative elasticity value corresponding to strain of tissue of the object can be obtained. However, the elastography image may be obtained at a relatively high frame rate, as compared to a shear wave elastography image from which an elasticity that is the absolute elasticity value of tissue of the object can be obtained.

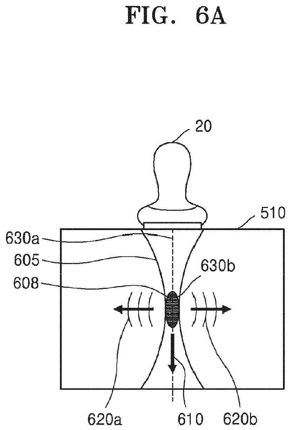

FIG. 6A illustrates a process of generating shear waves in an object according to an embodiment.

In an embodiment, a probe 20 may induce a displacement 610 of an object 510 by emitting a focus beam 605 onto the object 510. When the focus beam 605 is emitted onto the object 510, the displacement 610 may be induced in the object 510 at a focusing position 608 where the focus beam 605 is focused. Due to the displacement 610 of the object 510, shear waves 620a and 620b propagating in a vertical direction of the displacement 610 are generated at a position where the displacement 610 occurs. The shear waves 620a and 620b generated at the focusing position 608 travel along the vertical direction of the displacement 610 and gradually attenuate and disappear. A mode in which shear waves in the object 510 are captured is referred to as a shear wave elasticity mode, and the shear wave elasticity mode may include a 2D shear wave elasticity measurement mode and a point shear wave elasticity measurement mode.

According to the above embodiments, it has been described that shear wave elasticity measurement data of an object is acquired in the point shear wave elasticity measurement mode. However, a method of acquiring shear wave elasticity measurement data is not limited thereto, and may include acquisition of shear wave elasticity measurement data in a 2D shear wave elasticity measurement mode.

As shown in FIG. 6A, according to an embodiment, the ultrasound diagnosis apparatus 300 may generate shear waves in the object 510 by emitting a focus beam at a focusing position 630b on a determined focus beam scan line 630a.

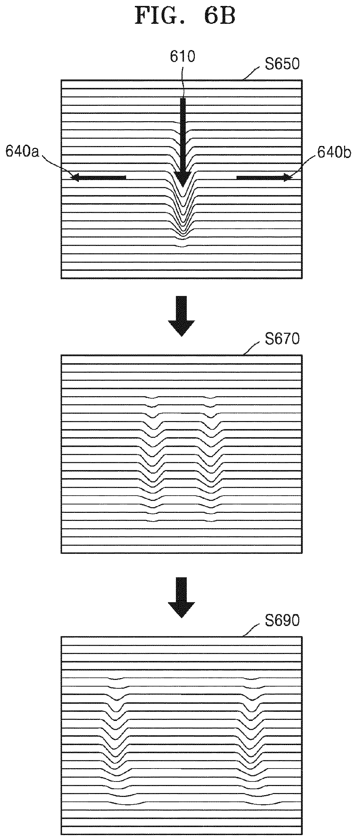

FIG. 6B illustrates propagation of shear waves according to an embodiment.

In an embodiment, as shown in operations S650, S670, and S690, shear waves generated due to emission of a focus beam by the probe 20 induce a displacement 610 at a focusing position and travel along directions indicated by arrows 640a and 640b.

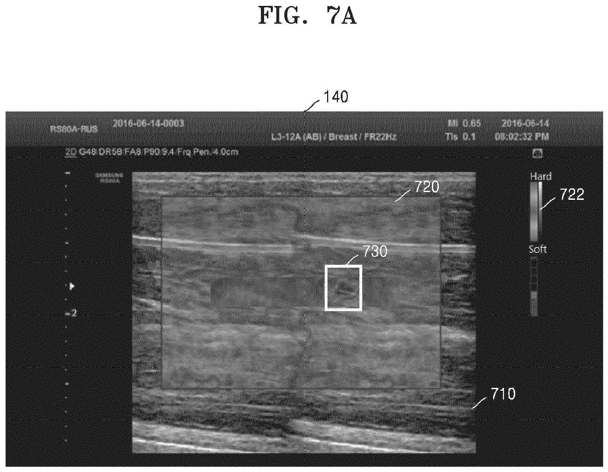

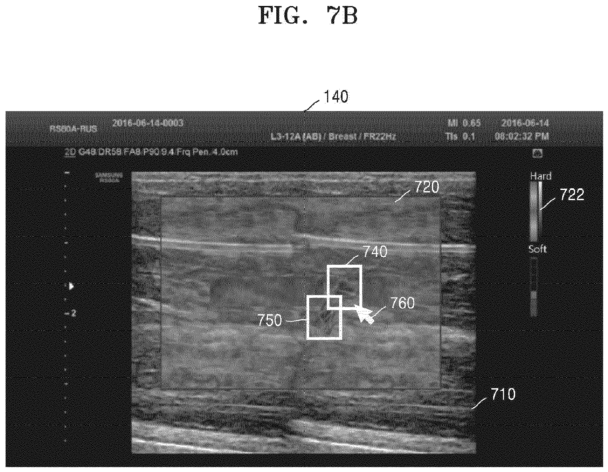

FIGS. 7A and 7B are images illustrating a process of determining an ROI for shear wave elasticity measurement based on elasticity data from an elastography image, according to an embodiment.

According to an embodiment, the ultrasound diagnosis apparatus 300 may display an elastography image 720 and a B-mode image 710 on the display 140 in such a manner that they overlap each other.

According to an embodiment, the display 140 of the ultrasound diagnosis apparatus 300 may display the elastography image 720 represented by a color bar 722 including colors that are distinguished according to elasticity values from the elastography image 720. For example, the display 140 of the ultrasound diagnosis apparatus 300 may show a point having a low elasticity value, such as hard tumor, in a red color and a point having a high elasticity value, such as relatively soft tissue, in a blue color.

According to an embodiment, the ultrasound diagnosis apparatus 300 may determine a first reference value that is an elasticity value used as a reference in determining an ROI. The ultrasound diagnosis apparatus 300 may determine, based on the first reference value, a region 730 in the elastography image 720 having an elasticity value less than the first reference value as being an ROI.

Referring to FIG. 7A, the region 730 determined as the ROI is indicated by the shape of a box, but a method of indicating the ROI is not limited thereto. The ROI may be displayed by using a graphical indicator including an arrow, a shape, text, etc., or a marker in such a manner that it is distinguished from other regions in the elastography image 720.

As shown in FIG. 7B, when there are a plurality of regions 740 and 750 having elasticity values less than a first reference value, the ultrasound diagnosis apparatus 300 may display the plurality of regions 740 and 750 in such a manner that they are distinguished from other regions in an elastography image 720.

According to an embodiment, the ultrasound diagnosis apparatus 300 may determine, as an ROI, a region selected according to a user input via a mouse cursor 760 or touch input via a touch screen or touch pad from among the plurality of regions 740 and 750 having elasticity values less than the first reference value. For example, referring to FIG. 7B, the user may perform an input for selecting the region 750 from among the plurality of regions 740 and 750 having the elasticity values less than the first reference value and displayed in such a manner as to be distinguished from other regions in the elastography image 720. Thereafter, the ultrasound diagnosis apparatus 300 may determine the region 740 selected according to the user input as an ROI for shear wave elasticity measurement.

In an embodiment, the ultrasound diagnosis apparatus 300 may determine one of the plurality of regions 740 and 750 having elasticity values less than the first reference value as an ROI for shear wave elasticity measurement according to a preset criterion. For example, the preset criterion may include determining a region including a point with the lowest elasticity value from among the plurality of regions 740 and 750 as the ROI or determining a region including the largest number of points with elasticity values less than the first reference value as the ROI. However, embodiments are not limited thereto, and the ultrasound diagnosis apparatus 300 may determine one of the plurality of regions 740 and 750 as an ROI according to various other criteria.

According to an embodiment, the ultrasound diagnosis apparatus 300 may receive a user input for selecting the region 730 as an ROI for shear wave elasticity measurement. For example, the user may select the region 730 indicated in red compared to other regions as the ROI, based on the elastography image 720 displayed on the display 140. The ultrasound diagnosis apparatus 300 may determine the selected region 730 as the ROI for shear wave elasticity measurement.

While FIGS. 7A and 7B show that the ultrasound diagnosis apparatus 300 displays the elastography image 720 as a 2D ultrasound image, embodiments are not limited thereto. According to an embodiment, the ultrasound diagnosis apparatus 300 may display the elastography image 720 as a 3D or 4D ultrasound image on the display 140. According to another embodiment, the ultrasound diagnosis apparatus 300 may display the elastography image 720 as a moving live image.

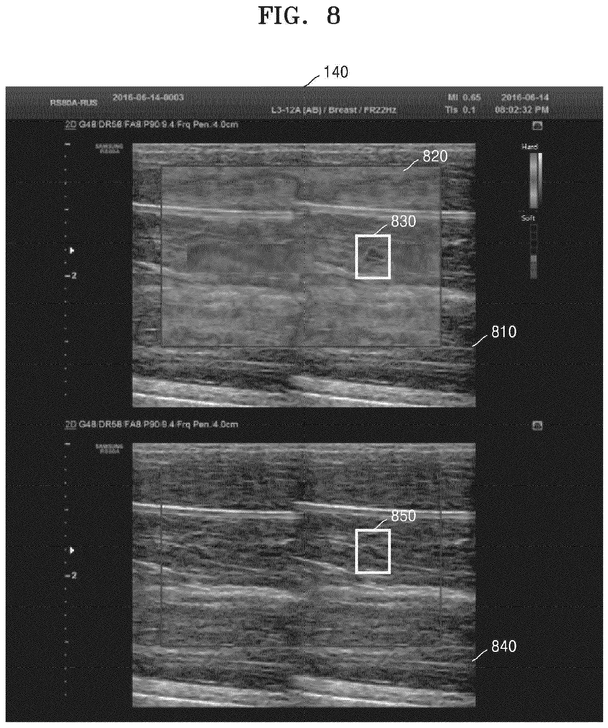

FIG. 8 illustrates a view showing an elastography image displayed on the display 140, according to an embodiment.

Referring to FIG. 8, the ultrasound diagnosis apparatus 300 may display a B-mode image 810 and an elastography image 820 overlapping each other in a first area of the display 140 and a B-mode image 840 in a second area thereof that is distinguished from the first area.

According to an embodiment, the ultrasound diagnosis apparatus 300 may indicate an ROI 850 corresponding to an ROI 830 in the elastography image 820 on the B-mode image 840 displayed in the second area. This may allow the user to determine which region in the B-mode image 840 showing a structure of an object corresponds to the ROI 830 determined based on the elastography image 820. While FIG. 8 shows that the first and second areas are respectively located at upper and lower parts of the display 140 in such a manner as to be distinguished from each other, embodiments are not limited thereto. For example, the first and second areas may be respectively located on left and right sides of the display 140 to be distinguished from each other.

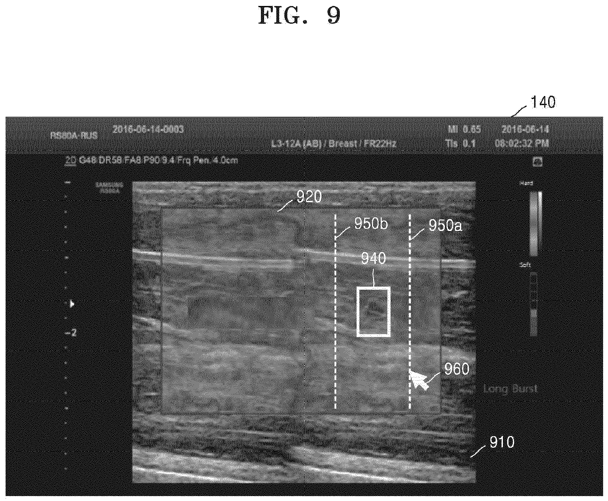

FIG. 9 illustrates a process of determining, based on an ROI, a focus beam scan line for shear wave elasticity measurement, according to an embodiment.

Referring to FIG. 9, according to an embodiment, the ultrasound diagnosis apparatus 300 may display a B-mode image 910 and an elastography image 920 of an object on the display 140 in such a manner that they overlap each other. Furthermore, the ultrasound diagnosis apparatus 300 may display a determined ROI 940 in such a manner as to distinguish it from other regions in the elastography image 920 based on an elasticity value obtained from the elastography image 920.

According to an embodiment, the ultrasound diagnosis apparatus 300 may determine a focus beam scan line for shear wave elasticity measurement based on the ROI 940. The ultrasound diagnosis apparatus 300 may determine two straight lines 950a and 950b that are respectively a first interval away from the ROI 940.

As shown in FIG. 9, according to an embodiment, the ultrasound diagnosis apparatus 300 may determine the two straight lines 950a and 950b that are respectively spaced rightward and leftward apart from the ROI 940 by the first interval, but embodiments are not limited thereto. The ultrasound diagnosis apparatus 300 may determine a plurality of scan lines, each having an interval that is within a first range away from the ROI 940.

According to an embodiment, the ultrasound diagnosis apparatus 300 may determine one of the determined two straight lines 950a and 950b as a focus beam scan line according to a preset criterion. For example, the ultrasound diagnosis apparatus 300 may determine the straight line 950a as a focus beam scan line according to a criterion that always determines a straight line located on the right side of the ROI 940 from among the determined two straight lines 950a and 950b as a focus beam scan line. According to an embodiment, the ultrasound diagnosis apparatus 300 may determine a focus beam scan line based on elasticity of regions respectively including the two straight lines 950a and 950b. For example, the ultrasound diagnosis apparatus 300 may determine a straight line included in a region having a low elasticity as a focus beam scan line. Furthermore, according to an embodiment, the ultrasound diagnosis apparatus 300 may determine the straight line 950a selected according to a user input via a mouse cursor 960 from among the determined two straight lines 950a and 950b as a focus beam scan line.

According to the above embodiments, the user may determine the ROI 940 where shear wave elasticity measurement data is to be acquired by examining the elastography image 920 displayed on the display 140, thereby accurately and conveniently determining the ROI 940 and a focusing position of a focus beam.

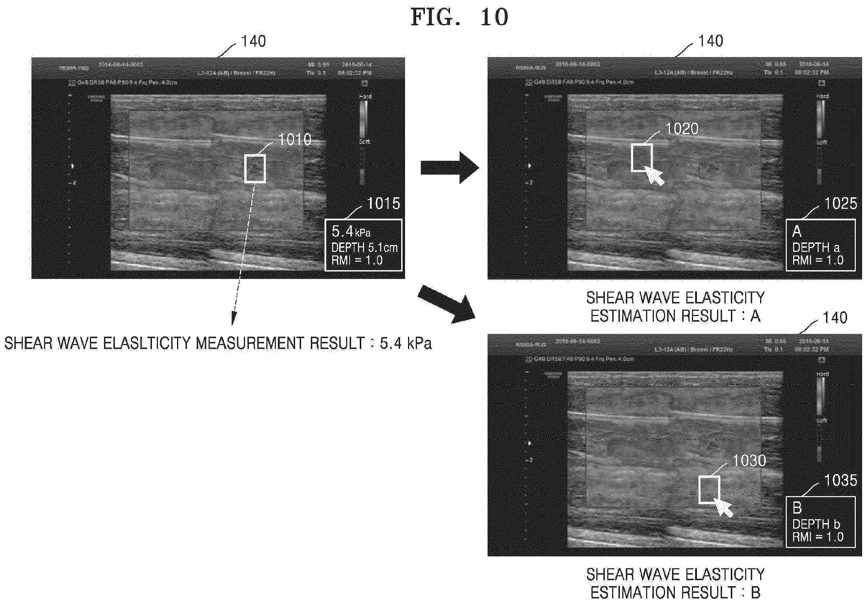

FIG. 10 illustrates a process of acquiring shear wave elasticity estimation data based on shear wave elasticity measurement data obtained via shear wave elasticity measurement.

According to an embodiment, the ultrasound diagnosis apparatus 300 may acquire shear wave elasticity measurement data with respect to a determined ROI 1010. Furthermore, the ultrasound diagnosis apparatus 300 may display the acquired shear wave elasticity measurement data in a first area 1015 of the display 140. For example, if a value of the shear wave elasticity measurement data obtained by the ultrasound diagnosis apparatus 300 at a depth of 5.1cm is 5.4 kilopascals (kPa), the ultrasound diagnosis apparatus 300 may display information indicating that the value of the shear wave elasticity measurement data obtained at the depth of 5.1cm is 5.4 kPa in the first region 1015 of the display 140.

In an embodiment, the ultrasound diagnosis apparatus 300 may estimate shear wave elasticity estimation data in regions other than the ROI 1010 based on the acquired shear wave elasticity measurement data. For example, if the user selects a region 1020, the ultrasound diagnosis apparatus 300 may obtain shear wave elasticity estimation data in the region 1020 based on an elasticity value obtained from an elastography image and the acquired shear wave elasticity measurement data. A value of shear wave elasticity estimation data may be calculated by using Equation (3) above, based on an elasticity value obtained from an elastography image and acquired shear wave elasticity measurement data in the ROI.

According to an embodiment, when a value of shear wave elasticity measurement data in a region 1020 having a depth of a, the ultrasound diagnosis apparatus 300 may display information indicating that the value of shear wave elasticity measurement data obtained at the depth of a is A in a first area 1025 of the display 140. Similarly, for example, if the user selects a region 1030 having a depth of b as a region where shear wave elasticity estimation data is to be obtained, the ultrasound diagnosis apparatus 300 may calculate a value of shear wave elasticity estimation data in the region 1030 as being B and display information indicating that the value of shear wave elasticity estimation data obtained at the depth of b is B in an area 1035 of the display 140.

According to an embodiment, the ultrasound diagnosis apparatus may acquire shear wave elasticity estimation data across the entire elastography image based on shear wave elasticity measurement data in an ROI. The ultrasound diagnosis apparatus 300 may obtain a shear wave elastography image based on shear wave elasticity measurement data and shear wave elasticity estimation data.

In an embodiment, the ultrasound diagnosis apparatus 300 may obtain a shear wave elastography image represented by a color bar including colors that are distinguished according to elasticity data values of the shear wave elastography image.

According to an embodiment, the ultrasound diagnosis apparatus 300 may display the obtained shear wave elastography image on the display 140.

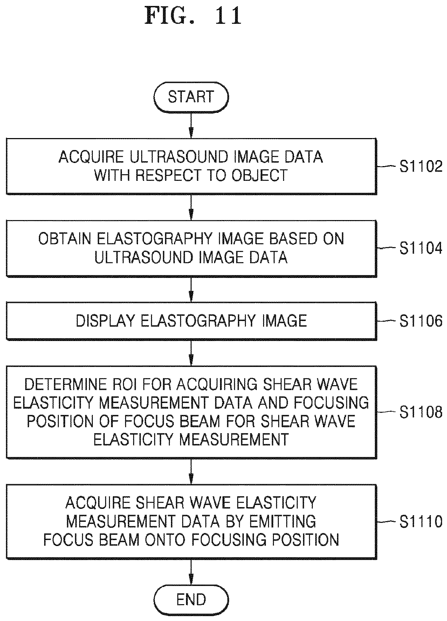

FIG. 11 is a flowchart of a method of controlling an ultrasound diagnosis apparatus, according to an embodiment.

The method according to the present embodiment may be performed by the ultrasound diagnosis apparatus

300 of FIG. 3. However, methods of controlling an ultrasound diagnosis apparatus according to embodiments may be performed by various ultrasound diagnosis apparatuses other than the ultrasound diagnosis apparatus 300 of FIG. 3.

The ultrasound diagnosis apparatus 300 acquires ultrasound image data with respect to an object by transmitting ultrasound signals to the object and detecting reflection signals corresponding to the transmitted ultrasound signals (S1102).

The ultrasound diagnosis apparatus 300 may acquire ultrasound image data with respect to the object by transmitting ultrasound signals to the object before and after compression is applied to the object and detecting reflected signals corresponding to the transmitted ultrasound signals.

The ultrasound diagnosis apparatus 300 obtains an elastography image based on the ultrasound image data (S1104).

For example, the ultrasound diagnosis apparatus 300 may calculate a displacement of tissue of the object from an obtained ultrasound image. The displacement may be calculated by comparing a plurality of ultrasound images obtained before and after compression is applied to the object.

The ultrasound diagnosis apparatus 300 calculates strain by differentiating the resulting displacement in a depth direction. Thereafter, the ultrasound diagnosis apparatus 300 may obtain an elastography image of the object based on the calculated strain.

The ultrasound diagnosis apparatus 300 may obtain, based on a strain of each tissue of the object, an elastography image represented by a color bar including colors that are distinguished according to elasticity values.

The ultrasound diagnosis apparatus 300 displays the obtained elastography image (S1106).

The ultrasound diagnosis apparatus 300 may display the obtained elastography image in such a manner as to overlay a B-mode image, but embodiments are not limited thereto. The ultrasound diagnosis apparatus 300 may display only an elastography image on the display 140 or both an elastography image and a B-mode image in separate areas of the display 140.

The ultrasound diagnosis apparatus 300 determines, based on the elastography image, an ROI for acquiring shear wave elasticity measurement data and a focusing position of a focus beam for performing shear wave elasticity measurement (S1108).

The ultrasound diagnosis apparatus 300 may determine an ROI for acquiring shear wave elasticity measurement data based on elasticity values obtained from the elastography image. For example, the ultrasound diagnosis apparatus 300 may determine a region in the elastography image, having an elasticity value less than a first reference value, as being an ROI. The first reference value may be acquired from a server (not shown) connected to the ultrasound diagnosis apparatus 300 by wire or wirelessly or may be a value prestored in the storage of the ultrasound diagnosis apparatus 300.

The ultrasound diagnosis apparatus 300 may determine a focus beam scan line for shear wave elasticity measurement based on an ROI and then a focusing position of a focus beam based on the determined focus beam scan line.

The ultrasound diagnosis apparatus 300 may obtain a first interval corresponding to an offset between the focusing position and the ROI, based on a variation in shear waves along a propagation direction. The first interval may be acquired from a server connected to the ultrasound diagnosis apparatus 300 by wire or wirelessly, may be input by the user, or may be a value prestored in the storage of the ultrasound diagnosis apparatus 300.

The ultrasound diagnosis apparatus 300 may determine a focus beam scan line having the first interval away from the ROI. For example, the ultrasound diagnosis apparatus 300 may determine a straight line in a depth direction, which is the first interval away from the ROI, as a focus beam scan line.

The ultrasound diagnosis apparatus 300 may determine a point on a focus beam scan line as a focusing position of a focus beam based on the ROI. For example, the ultrasound diagnosis apparatus 300 may determine a point on a focus beam scan line that is located at the same depth as the ROI as a focusing position of a focus beam. Furthermore, the ultrasound diagnosis apparatus 300 may determine, as the focusing position of the focus beam, one point from among a point on a focus beam scan line that is located at the same depth as the ROI and its adjacent points on the focus beam scan line.

The ultrasound diagnosis apparatus 300 emits a focus beam onto the determined focusing position and acquires shear wave elasticity measurement data (S1110).

According to an embodiment, the ultrasound diagnosis apparatus 300 may emit a focus beam onto the determined focusing position a plurality of times and acquire shear wave elasticity measurement data by averaging the resulting data.

The ultrasound diagnosis apparatus 300 may obtain shear wave elasticity estimation data in regions other than the ROI in the elastography image, based on the acquired shear wave elasticity measurement data and elasticity values obtained from the elastography image. The ultrasound diagnosis apparatus 300 may obtain a shear wave elastography image based on the shear wave elasticity measurement data and the shear wave elasticity estimation data.

According to the embodiments, by performing shear wave elasticity measurement, it is possible to automatically determine, based on an elastography image, an ROI where shear wave elasticity measurement data is to be acquired according to an elasticity value obtained from the elastography image Furthermore, the user may easily determine an ROI requiring shear wave elasticity measurement in an object based on a displayed elastography image and acquire shear wave elasticity measurement data in tissue where a lesion such as a tumor is located based on the determined ROI.

The above embodiments may be implemented through non-transitory computer-readable recording media having recorded thereon computer-executable instructions and data. The instructions may be stored in the form of program code, and when executed by a processor, generate a predetermined program module to perform a specific operation. Furthermore, when being executed by the processor, the instructions may perform specific operations according to the embodiments.

While one or more embodiments have been described with reference to the figures, it will be understood by those of ordinary skill in the art that various changes in form and details may be made therein without departing from the scope of the present disclosure as defined by the following claims. Accordingly, the above embodiments and all aspects thereof are examples only and are not limiting.

**Claims**

1. An ultrasound diagnosis apparatus comprising:

   an ultrasound probe configured to acquire ultrasound image data by transmitting ultrasound signals to an object and detecting reflected signals corresponding to the transmitted ultrasound signals;
   a processor configured to obtain an elastography image based on the ultrasound image data; and
   a display configured to display the elastography image,
   wherein the processor is further configured to determine, based on the elastography image, a region of interest (ROI) for acquiring shear wave elasticity measurement data and a focusing position of a focus beam for performing shear wave elasticity measurement, and
   wherein the ultrasound probe is further configured to emit the focus beam onto the focusing position and acquire shear wave elasticity measurement data.

2. The ultrasound diagnosis apparatus of claim 1, wherein the processor is further configured to calculate an elasticity value according to a position based on the elastography image and determine a region having the elasticity value less than a first reference value as being the ROI.

3. The ultrasound diagnosis apparatus of claim 2, further comprising a user input interface configured to receive, when there are a plurality of regions having elasticity values less than the first reference value, a user input for selecting one of the plurality of regions,
   wherein the processor is further configured to determine the ROI based on the user input.

4. The ultrasound diagnosis apparatus of any of the preceding claims, wherein the processor is further configured to determine a scan line for the focus beam, which has a first interval away from the ROI, and determine the focusing position of the focus beam based on the ROI.

5. The ultrasound diagnosis apparatus of any of the preceding claims, wherein the processor is further configured to determine a plurality of scan lines, each scan line having an interval that is within a first range away from the ROI, and wherein the display is further configured to display the determined plurality of scan lines,
   the ultrasound diagnosis apparatus, further comprising a user input interface configured to receive a user input for selecting one of the plurality of scan lines as a scan line for the focus beam.

6. The ultrasound diagnosis apparatus of any of the preceding claims, wherein the display is further configured to display the acquired shear wave elasticity measurement data.

7. The ultrasound diagnosis apparatus of claim 1, wherein the processor is further configured to obtain shear wave elasticity estimation data in a region other than the ROI in the elastography image based on elasticity data obtained from the elastography image and the shear wave elasticity measurement data acquired with respect to the ROI.

8. The ultrasound diagnosis apparatus of claim 7, wherein the display is further configured to display the shear wave elasticity measurement data and the shear wave elasticity estimation data.

9. The ultrasound diagnosis apparatus of any of the preceding claims, wherein the processor is further configured to determine a forbidden focus beam emission region based on the elastography image and determine the focusing position in such a manner as to prevent emission of the focus beam onto the forbidden focus beam emission region.

10. A method of controlling an ultrasound diagnosis apparatus, the method comprising:

acquiring ultrasound image data by transmitting ultrasound signals to an object and detecting reflected signals corresponding to the transmitted ultrasound signals;
obtaining an elastography image based on the ultrasound image data;
displaying the elastography image;
determining, based on the elastography image, a region of interest (ROI) for acquiring shear wave elasticity measurement data and a focusing position of a focus beam for performing shear wave elasticity measurement; and
emitting the focus beam onto the focusing position and acquiring shear wave elasticity measurement data.

11. The method of claim 10, wherein the determining of the ROI comprises calculating an elasticity value according to a position based on the elastography image and determining a region having the elasticity value less than a first reference value as being the ROI.

12. The method of claim 10 or 11, wherein the determining of the focusing position comprises:

determining a scan line for the focus beam, which has a first interval away from the ROI; and
determining the focusing position of the focus beam based on the ROI.

13. The method of claim 10, 11 or 12, further comprising displaying the acquired shear wave elasticity measurement data.

14. The method of any of claims 10-13, further comprising obtaining shear wave elasticity estimation data in a region other than the ROI in the elastography image based on elasticity data obtained from the elastography image and the shear wave elasticity measurement data acquired with respect to the ROI.

15. A non-transitory computer-readable recording medium having recorded thereon a program for executing the method of any of claims 10-14 on a computer.

## FIG. 1

FIG. 2A

FIG. 2B

FIG. 2C

# FIG. 3

# FIG. 4

# FIG. 5

(S502)

(S504)

(S506)

# FIG. 6A

# FIG. 6B

FIG. 7A

FIG. 7B

# FIG. 8

FIG. 9

**FIG. 10**

# FIG. 11

```
                        ( START )
                            │
                            ▼
          ┌─────────────────────────────────┐
          │   ACQUIRE ULTRASOUND IMAGE DATA  │
          │       WITH RESPECT TO OBJECT      │──── S1102
          └─────────────────────────────────┘
                            │
                            ▼
          ┌─────────────────────────────────┐
          │ OBTAIN ELASTOGRAPHY IMAGE BASED ON│
          │       ULTRASOUND IMAGE DATA      │──── S1104
          └─────────────────────────────────┘
                            │
                            ▼
          ┌─────────────────────────────────┐
          │    DISPLAY ELASTOGRAPHY IMAGE    │──── S1106
          └─────────────────────────────────┘
                            │
                            ▼
          ┌─────────────────────────────────┐
          │ DETERMINE ROI FOR ACQUIRING SHEAR WAVE │
          │ ELASTICITY MEASUREMENT DATA AND FOCUSING │──── S1108
          │  POSITION OF FOCUS BEAM FOR SHEAR WAVE  │
          │       ELASTICITY MEASUREMENT     │
          └─────────────────────────────────┘
                            │
                            ▼
          ┌─────────────────────────────────┐
          │   ACQUIRE SHEAR WAVE ELASTICITY  │
          │      MEASUREMENT DATA BY EMITTING │──── S1110
          │  FOCUS BEAM ONTO FOCUSING POSITION│
          └─────────────────────────────────┘
                            │
                            ▼
                         ( END )
```

Europäisches
Patentamt

European
Patent Office

Office européen
des brevets

# EUROPEAN SEARCH REPORT

Application Number

EP 17 16 9053

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2015/148674 A1 (PARK JI-YOUNG [KR] ET AL) 28 May 2015 (2015-05-28) * abstract; figures 1, 2A-B, 5-14 * * paragraphs [0066] - [0072], [0096] - [0158] * | 1-15 | INV. A61B8/08 |
| X | US 2015/164476 A1 (KONG DONG-GEON [KR] ET AL) 18 June 2015 (2015-06-18) * abstract; figures 1-5 * * paragraphs [0044] - [0074] * | 1-15 | |
| X | US 2013/317361 A1 (TABARU MARIE [JP] ET AL) 28 November 2013 (2013-11-28) * abstract; figures 2-14 * * paragraphs [0048] - [0059] * | 1-15 | |
| A | US 2016/143621 A1 (PARTHASARATHY VIJAY [US] ET AL) 26 May 2016 (2016-05-26) * abstract; figures 4-8, 11 * * paragraphs [0051] - [0060] * | 1-15 | |
| | | | TECHNICAL FIELDS SEARCHED (IPC) |
| | | | A61B |

The present search report has been drawn up for all claims

| Place of search | Date of completion of the search | Examiner |
|---|---|---|
| Munich | 12 December 2017 | Lahorte, Philippe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

             

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 17 16 9053

This annex lists the patent family members relating to the patent documents cited in the above-mentioned European search report.
The members are as contained in the European Patent Office EDP file on
The European Patent Office is in no way liable for these particulars which are merely given for the purpose of information.

12-12-2017

| Patent document cited in search report | | Publication date | Patent family member(s) | | Publication date |
|---|---|---|---|---|---|
| US 2015148674 | A1 | 28-05-2015 | EP | 2878270 A2 | 03-06-2015 |
| | | | KR | 20150061989 A | 05-06-2015 |
| | | | US | 2015148674 A1 | 28-05-2015 |
| US 2015164476 | A1 | 18-06-2015 | CN | 104706384 A | 17-06-2015 |
| | | | KR | 20150070859 A | 25-06-2015 |
| | | | US | 2015164476 A1 | 18-06-2015 |
| US 2013317361 | A1 | 28-11-2013 | CN | 103347450 A | 09-10-2013 |
| | | | EP | 2671511 A1 | 11-12-2013 |
| | | | JP | 5619191 B2 | 05-11-2014 |
| | | | JP | WO2012105152 A1 | 03-07-2014 |
| | | | US | 2013317361 A1 | 28-11-2013 |
| | | | WO | 2012105152 A1 | 09-08-2012 |
| US 2016143621 | A1 | 26-05-2016 | CN | 105491959 A | 13-04-2016 |
| | | | EP | 3013243 A1 | 04-05-2016 |
| | | | JP | 2016523167 A | 08-08-2016 |
| | | | RU | 2016102134 A | 27-07-2017 |
| | | | US | 2016143621 A1 | 26-05-2016 |
| | | | WO | 2014207668 A1 | 31-12-2014 |

EPO FORM P0459

For more details about this annex : see Official Journal of the European Patent Office, No. 12/82